Europäisches Patentamt

European Patent Office

Office européen des brevets

(19)

(11) Veröffentlichungsnummer: **0 174 597**
**B1**

(12) **EUROPÄISCHE PATENTSCHRIFT**

(45) Veröffentlichungstag der Patentschrift:
**07.12.88**

(21) Anmeldenummer: **85111145.0**

(22) Anmeldetag: **04.09.85**

(51) Int. Cl.⁴: $C\ 07\ D\ 333/08$

(54) **Verfahren zur Isomerisierung von Alkylthiophenen.**

(30) Priorität: **13.09.84 DE 3433813**

(43) Veröffentlichungstag der Anmeldung:
**19.03.86 Patentblatt 86/12**

(45) Bekanntmachung des Hinweises auf die Patenterteilung:
**07.12.88 Patentblatt 88/49**

(84) Benannte Vertragsstaaten:
**BE CH DE FR GB IT LI NL**

(56) Entgegenhaltungen:
**GB-A- 860 673**

(73) Patentinhaber: **HOECHST AKTIENGESELLSCHAFT,
Postfach 80 03 20, D-6230 Frankfurt am Main 80 (DE)**

(72) Erfinder: **Eichler, Klaus, Dr., im Traminer 10,
D-6236 Eschborn 2 (DE)**
Erfinder: **Leupold, Ernst Ingo, Dr., Am Zäunefeld 15,
D-6392 Neu-Anspach (DE)**

## Beschreibung

Die vorliegende Erfindung betrifft ein Verfahren zur Isomerisierung von Alkylthiophenen.

Alkylierte Thiophene sind wertvolle Vorprodukte. So dienen zum Beispiel die Methylthiophene als Ausgangsstoffe für die Herstellung von Thenylchlorid und Thiophencarbonsäure, die bei der Herstellung von Pharmazeutika benötigt werden (Ullmanns Enzyklopädie der technischen Chemie; 4. Auflage, Band 23. Seite 219). Sie werden im allgemeinen durch Cyclisierungsreaktionen von Olefinen oder Alkoholen mit $H_2S$ oder $CS_2$ hergestellt (GB-PS 1 345 203, DE-OS 2 757 816). Die Synthese der 3-substituierten Alkylthiophene erfordert dabei verzweigte Ausgangsstoffe, die zum Teil sehr teuer sind oder nicht zur Verfügung stehen. Einfache und preiswerte Methoden zur Herstellung von 3-Alkylthiophenen im technischen Massstab wurden bisher nicht beschrieben.

GB-A-860 673 beschreibt die Umsetzung von Alkylthiophenen an einem HF-haltigen Aluminiumoxid-Katalysator. Dabei tritt im wesentlichen Dealkylierung, Disproportionierung und Aromatisierung ein. Eine wesentliche Isomerisierung wurde nicht beobachtet.

Es wurde nun gefunden, dass sich alkylierte Thiophene mit hoher Ausbeute an Zeolith-Katalysatoren isomerisieren lassen.

Gegenstand der Erfindung ist daher ein Verfahren zur Isomerisierung von Alkylthiophenen, dadurch gekennzeichnet, dass man ein Alkylthiophen oder ein Gemisch aus Alkylthiophenen mit einem Zeolith-Katalysator in Berührung bringt. Insbesondere ist Gegenstand der Erfindung ein Verfahren zur Isomerisierung von Methyl- oder Ethylthiophen an einem Zeolith-Katalysator. Der Begriff «Alkylthiophene» soll alle Thiophene umfassen, die ein bis drei geradkettige oder verzweigte Alkylgruppen mit ein bis sechs C-Atomen enthalten, also zum Beispiel Methyl-, Ethyl-, Isopropyl-, Dimethyl-, Diethyl- oder Di-t-butylthiophen.

Aufgrund des Standes der Technik war es überraschend und in keiner Weise vorhersehbar, dass sich Alkylthiophene in so einfacher Weise mit so hohen Ausbeuten, wie die Beispiele zeigen, isomerisieren lassen und dass sich bisher schwer herstellbare 3-Alkylthiophene so leicht aus technisch zugänglichen 2-Alkylthiophenen herstellen lassen.

Beispiel 1 zeigt, dass sich 2-Methylthiophen an H-ZSM-5 mit guter Ausbeute zu 3-Methylthiophen isomerisieren lässt. Dabei treten kaum Nebenprodukte auf. Noch besser gelingt die Isomerisierung bei höherer Temperatur (Beispiel 2). Wie die Isomerisierung von 3-Methylthiophen zu 2-Methylthiophen zeigt (Beispiel 3), wird unabhängig vom Ausgangsmaterial das thermodynamische Gleichgewicht erreicht. Aus Beispiel 4 ist ersichtlich, dass sich 2-Ethylthiophen bereits bei 300 °C mit hoher Ausbeute zu 3-Ethylthiophen isomerisieren lässt.

Zur Durchführung des erfindungsgemässen Verfahrens wird ein Alkylthiophen oder ein Gemisch aus zwei oder mehr Alkylthiophenen mit dem Zeolith-Katalysator in Kontakt gebracht.

Als Zeolithe eignen sich im allgemeinen sowohl natürliche wie auch synthetische Zeolithe, vorzugsweise synthetische Zeolithe vom Pentasil-, Mordenit- oder Faujasit-Typ, insbesondere synthetische Zeolithe vom Pentasil-Typ.

Für den Begriff Pentasile gilt dabei die Definition von Kokotailo und Meier («Pentasil family of high silicon crystalline materials» in Special Publication No. 33 of the Chemical Society, London, 1980). Die Pentasil-Familie umfasst beispielsweise die synthetischen Zeolithe ZSM-5 (US-PS 3 702 886), ZMS-8 (GB-PS 1 334 243); ZMS-11 (US-PS 3 709 979) und ZMS-23 (US-PS 4 076 842).

Das Si/Al-Verhältnis der Pentasile liegt vorzugsweise bei 20 bis 2000, das der Mordenite bei vorzugsweise 5 bis 100. Pentasile oder Mordenite mit einem höheren Aluminiumgehalt lassen sich dabei auf das gewünschte Si/Al-Verhältnis einstellen, indem man durch Behandlung mit Mineralsäuren, organischen Säuren oder chelatisierenden Substanzen einen Teil des Aluminiums aus dem Zeolith-Gitter entfernt.

Bei dem erfindungsgemässen Verfahren werden die Zeolithe vorzugsweise in ihrer sauren Form eingesetzt. Diese sauren Formen lassen sich nach bekannten Methoden aus den Alkalimetall-Formen, wie sie in der Regel bei der Zeolith-Synthese anfallen bzw. als Naturprodukte vorkommen, durch vollständigen oder partiellen Ionenaustausch herstellen. Eine übliche Methode zur Herstellung der H-Form eines Zeoliths besteht beispielsweise darin, die Alkalimetall-Form zunächst durch partiellen oder vollständigen Ionenaustausch mit einer Ammoniumsalz-Lösung in die Ammoniumform und diese anschliessend durch Kalzinieren in die H-Form zu überführen. Aber auch die mit Alkali-, Erdalkali- und mit Seltenerdmetall-Ionen ausgetauschten Formen zeigen katalytische Aktivität.

Für das erfindungsgemässe Verfahren sind auch Zeolithe geeignet, bei denen Aluminium- oder Siliziumatome durch andere Gitteratome wie Bor, Eisen, Gallium, Germanium, Titan oder Zirkon ersetzt sind.

Die erfindungsgemässen Zeolith-Katalysatoren bestehen im allgemeinen aus der katalytisch aktiven Zeolith-Komponente sowie einem Bindermaterial. Letzteres ist erforderlich, um den Zeolith in eine für das erfindungsgemässe Verfahren geeignete äussere Form zu bringen.

Als Bindermaterial eignen sich vor allem Oxide oder Hydroxide des Aluminiums und die Oxide bzw. Hydroxide des Siliciums, sowie Schichtsilicate, beispielsweise aus der Kaolin- oder Montmorillonit-Familie.

Dieser so hergestellte Zeolith-Katalysator wird gewöhnlich vor dem Einsatz in der erfindungsgemässen Isomerisierungsreaktion zunächst durch Kalzinieren bei Temperaturen zwischen 300 und 700 °C aktiviert. Zur besseren Stabilisierung des Katalysators ist es manchmal vorteilhaft, die Kalzinierung in Gegenwart von Wasserdampf, Ammoniak oder deren Mischungen durchzuführen.

Falls in der Gasphase gearbeitet werden soll, besteht eine günstige einfache Verfahrensweise zur Durchführung der erfindungsgemässen Isomerisierung darin, dass man das Alkylthiophen oder die Alkylthiophene aus einer Dosiervorrichtung zuerst in eine Verdampfungszone und das entstandene Gas dann durch ein von aussen beheiztes und mit dem Katalysator gefülltes Reaktionsrohr leitet. Bei Durchführung der Isomerisierung in flüssiger Phase werden das oder die Thiophene, ggf. verdünnt, erst erwärmt und dann in flüssiger Form durch das mit dem Katalysator gefüllte Reaktionsrohr geleitet.

In der Verdampfungs- oder Erwärmungszone erfolgt gegebenenfalls noch eine Vermischung mit Wasserstoff, Stickstoff und/oder einem anderen Trägergas, wobei Wasserstoff bevorzugt wird. Es hat sich dabei als vorteilhaft erwiesen, diese Gase vor der Vermischung auf Reaktionstemperatur aufzuheizen.

Die Belastung des Zeolith-Katalysators — ausgedrückt als LHSV (Liquid Hourly Space Velocity, $h^{-1}$) — liegt dabei im allgemeinen zwischen 0,05 und 10 $h^{-1}$, vorzugsweise zwischen 0,1 und 5 $h^{-1}$.

Die erfindungsgemässe Isomerisierung wird im allgemeinen bei Temperaturen zwischen 150 und 550 °C, vorzugsweise bei 200 bis 450 °C, sowie Drücken von 0,1 bis 10 bar, vorzugsweise bei Normaldruck, durchgeführt.

Die Reaktionsprodukte werden nach Verlassen des Reaktors zur Abtrennung der kondensierbaren Anteile gekühlt. Die erfindungsgemässe Isomerisierung ist jedoch nicht auf diese Verfahrensweise (Festbett-Reaktor) beschränkt, sondern lässt sich im Prinzip auch in anderen geeigneten Reaktor-Typen durchführen (z.B. Wirbelschicht-Reaktor).

Das entstandene Isomerengemisch kann nach bekannten Verfahren destillativ getrennt werden. Das nicht umgesetzte Ausgangsprodukt kann in den Reaktor zurückgeführt werden.

Falls die Aktivität des Katalysators langsam aufgrund einer Verkokung abnimmt, kann er von Zeit zu Zeit regeneriert werden. Dies geschieht, indem Sauerstoff, Luft, Stickstoff/Luft, Sauerstoff/Luft, Sauerstoff/Inertgas oder Luft/Inertgas bei Temperaturen zwischen 300 und 650 °C über den desaktivierten Katalysator geleitet wird. Stickstoff/Luft wird dabei bevorzugt. Die Temperatur sollte dabei an keiner Stelle des Reaktors 650 °C übersteigen.

Die Erfindung soll durch die folgenden Beispiele erläutert werden.

Beispiele
Herstellung des Katalysators

100 g ZSM-5-Pulver in der Na-Form (US-PS 3 702 886, Beispiel 1) wurden bei 100 °C dreimal für 5 Stunden mit 1-molarer Ammoniumchloridlösung behandelt, gewaschen, getrocknet und 5 Stunden bei 550 °C in Luft calciniert. 65 g des erhaltenen Pulvers wurden mit 35 g $Al_2O_3$ zu Strangpresslingen von 1,6 mm Durchmesser verarbeitet, 4 h bei 500 °C calciniert, auf eine Teilchengrösse von 0,25 bis 1,0 mm zerkleinert und bei 450 °C im Stickstoffstrom zwei Stunden lang calciniert.

Beschreibung der Apparatur

15 ml des oben beschriebenen Katalysators wurden in einen Rohrreaktor aus Glas von 16 mm Innendurchmesser und 50 cm Länge eingefüllt und mit Glaskugeln (zum Verdampfen der flüssigen Reaktanden) überschichtet. Der Reaktor befand sich in einem elektrisch beheizten Ofen. Flüssige Reaktanden wurden über eine Dosierpumpe zugeführt, Gase über eine Gasversorgung, bestehend aus Reduzierventilen und Vorrichtungen zum Messen des Drucks und der Durchflussmenge. Die kondensierbaren Reaktionsprodukte wurden in einer Kühlfalle bei 0 °C kondensiert und gaschromatographisch analysiert.

Beispiel 1
Isomerisierung von 2-Methylthiophen

6 ml/h 2-Methylthiophen wurden zusammen mit 4,5 l/h Wasserstoff über den oben beschriebenen Katalysator geleitet. Nach einer kurzen Vorlaufzeit von etwa 15 Minuten zur Einstellung konstanter Reaktionsbedingungen wurde stündlich die Vorlage gewechselt und anschliessend analysiert. Tabelle 1 zeigt die Ergebnisse.

Tabelle 1
Isomerisierung von 2-Methylthiophen

| Dauer (h) | Temperatur (°C) | Produktzusammensetzung | | | |
|---|---|---|---|---|---|
| | | Thiophen (Gew.-%) | 2-Methylthiophen (Gew.-%) | 3-Methylthiophen (Gew.-%) | Dimethylthiophene (Gew.-%) |
| 1 | 200 | 0,1 | 98,5 | 0,2 | 0,1 |
| 2 | 200 | 0,1 | 99,1 | 0,1 | 0,2 |
| 3 | 230 | 0,1 | 98,4 | 0,7 | 0,2 |
| 4 | 260 | 0,5 | 89,7 | 8,0 | 0,6 |
| 5 | 260 | 0,2 | 89,4 | 9,6 | 0,3 |
| 6 | 300 | 0,7 | 70,0 | 26,9 | 1,4 |
| 7 | 300 | 0,5 | 77,5 | 20,4 | 1,0 |

Tabelle 1 (Fortsetzung)
Isomerisierung von 2-Methylthiophen

| Dauer (h) | Tempe-ratur (°C) | Produktzusammensetzung | | | |
|---|---|---|---|---|---|
| | | Thiophen (Gew.-%) | 2-Methyl-thiophen (Gew.-%) | 3-Methyl-thiophen (Gew.-%) | Dimethyl-thiophene (Gew.-%) |
| 8 | 330 | 0,9 | 53,3 | 42,3 | 1,6 |
| 9 | 330 | 0,6 | 58,2 | 39,5 | 1,4 |
| 10 | 330 | 0,5 | 62,2 | 36,0 | 1,0 |
| 15 | 330 | 0,3 | 71,2 | 26,2 | 1,7 |
| 20 | 330 | 0,3 | 76,8 | 22,2 | 0,4 |
| 25 | 330 | 0,2 | 84,6 | 14,8 | 0,3 |
| 30 | 330 | 0,2 | 89,2 | 10,0 | 0,3 |

Beispiel 2
(Isomerisierung von 2-Methylthiophen)

6 ml/h 2-Methylthiphen wurden zusammen mit 4,5 l/h Wasserstoff über den oben beschriebenen Katalysator geleitet. Nach einer kurzen Vorlaufzeit von 15 Minuten zur Einstellung konstanter Reaktionsbedingungen wurde stündlich die Vorlage gewechselt und analysiert. Tabelle 2 zeigt die Ergebnisse.

Tabelle 2
Isomerisierung von 2-Methylthiophen

| Dauer (h) | Tempe-ratur (°C) | Produktzusammensetzung | | | |
|---|---|---|---|---|---|
| | | Thiophen (Gew.-%) | 2-Methyl-thiophen (Gew.-%) | 3-Methyl-thiophen (Gew.-%) | Dimethyl-thiophene (Gew.-%) |
| 1 | 300 | 1,5 | 53,9 | 40,0 | 3,6 |
| 2 | 300 | 0,6 | 68,6 | 29,3 | 1,1 |
| 3 | 300 | 0,5 | 78,9 | 19,3 | 1,0 |
| 4 | 300 | 0,4 | 80,9 | 17,6 | 0,8 |
| 5 | 330 | 0,8 | 57,6 | 39,6 | 1,7 |
| 8 | 330 | 0,4 | 69,3 | 29,7 | 0,6 |
| 9 | 360 | 0,7 | 51,6 | 46,0 | 1,3 |
| 12 | 360 | 0,6 | 52,8 | 44,6 | 0,8 |
| 13 | 390 | 1,2 | 46,9 | 49,3 | 1,9 |
| 16 | 390 | 0,8 | 48,7 | 48,5 | 1,4 |
| 17 | 420 | 2,0 | 46,7 | 47,3 | 2,7 |
| 20 | 420 | 1,1 | 47,7 | 48,0 | 1,1 |
| 23 | 420 | 0,8 | 48,6 | 48,9 | 1,1 |
| 25 | 450 | 2,0 | 44,7 | 47,3 | 2,3 |
| 29 | 450 | 1,0 | 45,2 | 48,9 | 1,4 |
| 33 | 450 | 0,8 | 48,0 | 52,1 | 1,1 |
| 39 | 450 | 0,7 | 45,8 | 51,8 | 0,9 |

Beispiel 3
(Isomerisierung von 3-Methylthiophen)

Der im Beispiel 2 beschriebene Versuch wurde fortgesetzt, indem statt des 2-Methylthiophens ab der 39. Stunde 3-Methylthiophen eingesetzt wurde. Tabelle 3 zeigt die Ergebnisse.

Tabelle 3
Isomerisierung von 3-Methylthiophen

| Dauer (h) | Tempe-ratur (°C) | Produktzusammensetzung | | | |
|---|---|---|---|---|---|
| | | Thiophen (Gew.-%) | 2-Methyl-thiophen (Gew.-%) | 3-Methyl-thiophen (Gew.-%) | Dimethyl-thiophene (Gew.-%) |
| 40 | 450 | 0,5 | 43,2 | 54,4 | 0,8 |
| 41 | 450 | 0,5 | 49,2 | 49,1 | 0,8 |
| 42 | 450 | 0,5 | 46,1 | 52,3 | 0,7 |
| 43 | 450 | 0,5 | 48,2 | 49,7 | 1,0 |

Beispiel 4
(Isomerisierung von 2-Ethylthiophen)

6 ml/h 2-Ethylthiophen und 4,5 l/h Wasserstoff wurden über den in Beispiel 1 beschriebenen Katalysator geleitet. Nach einer kurzen Vorlaufzeit von etwa 15 Minuten zur Einstellung konstanter Reaktionsbedingungen wurde stündlich die Vorlage gewechselt und anschliessend analysiert. Tabelle 4 zeigt die Ergebnisse.

Tabelle 4
Isomerisierung von 2-Ethylthiophen

| Dauer (h) | Tempe-ratur (°C) | Produktzusammensetzung | | | |
|---|---|---|---|---|---|
| | | Thiophen (Gew.-%) | 2-Ethyl-thiophen (Gew.-%) | 3-Ethyl-thiophen (Gew.-%) | Diethyl-thiophene (Gew.-%) |
| 1 | 300 | 2,0 | 46,3 | 40,3 | 4,2 |
| 3 | 300 | 0,7 | 51,4 | 45,2 | 1,0 |
| 5 | 300 | 0,6 | 53,2 | 44,2 | 0,7 |
| 7 | 300 | 0,4 | 57,6 | 40,3 | 0,9 |

## Patentansprüche

1. Verfahren zur Isomerisierung von Alkylthiophenen, dadurch gekennzeichnet, dass man ein Alkylthiophen oder ein Gemisch aus Alkylthiophenen mit einem Zeolith-Katalysator in Berührung bringt.

2. Verfahren nach Anspruch 1, dadurch gekennzeichnet, dass man Methylthiophen oder Ethylthiophen einsetzt.

3. Verfahren nach einem der Ansprüche 1 oder 2, dadurch gekennzeichnet, dass man einen Zeolith vom Pentasil-, Mordenit- oder Faujasit-Typ einsetzt.

4. Verfahren nach einem der Ansprüche 1 bis 3, dadurch gekennzeichnet, dass man einen sauren Zeolith vom Pentasil-, Mordenit- oder Faujasit-Typ einsetzt.

5. Verfahren nach einem der Ansprüche 1 bis 3, dadurch gekennzeichnet, dass man einen sauren Zeolith vom Pentasil-Typ einsetzt.

6. Verfahren nach Anspruch 4 oder 5, dadurch gekennzeichnet, dass der saure Zeolith Protonen als Kationen enthält.

7. Verfahren nach einem der Ansprüche 1 bis 6, dadurch gekennzeichnet, dass man bei Temperaturen zwischen 150 und 550 °C arbeitet.

8. Verfahren nach einem der Ansprüche 1 bis 7, dadurch gekennzeichnet, dass man bei einem Druck zwischen 0,1 bar und 10 bar arbeitet.

9. Verfahren nach einem der Ansprüche 1 bis 8, dadurch gekennzeichnet, dass man in Gegenwart von Wasserstoff, Stickstoff, Wasserdampf, Argon oder einem Gemisch aus diesen arbeitet.

## Revendications

1. Procédé pour isomériser des alkyl-thiophènes, caractérisé en ce qu'on met un alkyl-thiophène, ou un mélange d'alkyl-thiophènes, en contact avec un catalyseur à base de zéolites.

2. Procédé selon la revendication 1 caractérisé en ce qu'on utilise un méthyl-thiophène ou un éthylthiophène.

3. Procédé selon l'une des revendications 1 et 2, caractérisé en ce qu'on utilise une zéolite du genre du pentasil, de la mordénite ou de la faujasite.

4. Procédé selon l'une quelconque des revendications 1 à 3, caractérisé en ce qu'on utilise une zéolite acide du genre du pentasil, de la mordénite ou de la faujasite.

5. Procédé selon l'une quelconque des revendications 1 à 3, caractérisé en ce qu'on utilise une zéolite acide du type du pentasil.

6. Procédé selon l'une des revendications 4 et 5, caractérisé en ce que la zéolite acide contient des protons comme cations.

7. Procédé selon l'une quelconque des revendications 1 à 6, caractérisé en ce qu'on opère à des températures comprises entre 150 et 550 °C.

8. Procédé selon l'une quelconque des revendications 1 à 7, caractérisé en ce qu'on opère sous une pression de 0,1 et 10 bar.

9. Procédé selon l'une quelconque des revendications 1 à 8, caractérisé en ce qu'on opère en présence d'hydrogène, d'azote, de vapeur d'eau, d'argon ou d'un mélange de ces corps.

## Claims

1. A process for isomerizing alkylthiophenes, which comprises contacting an alkylthiophene or a mixture of alkylthiophenes with a zeolite catalyst.

2. The process as claimed in claim 1, wherein methylthiophene or ethylthiophene is employed.

3. The process as claimed in claim 1 or 2, wherein a zeolite of the pentasil, mordenite or faujasite type is employed.

4. The process as claimed in any of claims 1 to 3, wherein an acid zeolite of the pentasil, mordenite or faujasite type is employed.

5. The process as claimed in any of claims 1 to 3, wherein an acid zeolite of the pentasil type is employed.

6. The process as claimed in claim 4 or 5, wherein the acid zeolite contains protons as the cations.

7. The process as claimed in any of claims 1 to 6, when carried out at temperatures between 150 and 550 °C.

8. The process as claimed in any of claims 1 to 7, when carried out under a pressure between 0.1 bar and 10 bar.

9. The process as claimed in any of claims 1 to 8, when carried out in the presence of hydrogen, nitrogen, steam, argon or a mixture thereof.